# EUROPEAN PATENT SPECIFICATION

(11) **EP 1 911 455 B1**
(45) Date of publication and mention of the grant of the patent: **12.06.2013**
(21) Application number: 07019269.5
(22) Date of filing: 01.10.2007
(51) Int. Cl.: A61K 35/74, A61K 31/19, A61K 36/8962, A61K 9/20, A61K 9/28

(54) **Topical vaginal pharmaceutical compositions**
Topische pharmazeutische Zusammensetzungen für die Vagina
Compositions pharmaceutiques vaginales topiques

(30) Priority: 04.10.2006 IT mi20061912
(43) Date of publication of application: 16.04.2008
(73) Proprietor: S.I.I.T. S.r.l.-Servizio Internazionale Imballaggi Termosaldanti, 20090 Trezzano sul Naviglio MI (IT)
(72) Inventor: Di Pierro, Francesco, 20090 Trezzano S/N (MI) (IT)
(74) Representative: Minoja, Fabrizio

(56) References cited:
- EP-A- 1 481 666
- WO-A-03/092650
- US-A1- 2005 208 034

## Description

This invention relates to topical vaginal pharmaceutical formulations with a local action based on lactobacilli, lactic acid and extracts of garlic and/or derivatives/fractions thereof.

### PRIOR ART

Under normal conditions, the vaginal mucosa is inhabited by a rich, varied flora of micro-organisms, which are very often tissue-specific. This microbial flora can be conveniently divided into two categories: "resident" flora, which is almost invariably present and, if altered, can be restored; and "transient" flora, which can colonise the host for short periods, but tends to be eliminated by competition from the resident micro-organisms or the host' s defence mechanisms. The transient flora can also consist of potentially pathogenic micro-organisms, but this is not very common in healthy individuals.

Normal vaginal flora represents an effective barrier that reduces the probability of colonisation of the host surfaces by pathogenic micro-organisms (a phenomenon known as "colonisation-resistance").

In adults, antibiotic treatment, stress, dietary imbalance and disease alter the quality and quantity of the beneficial microbial vaginal flora, thus exposing the vaginal mucosa to potential infections. The problem of achieving a fast, efficient recolonisation process consequently arises. This process is significantly facilitated by "probiotics". These products, normally used as nutritional supplements in the prophylaxis of gastroenteric disorders, can also be used "topically", ie. applied directly to the mucosa to prevent vaginal infections.

Probiotics are live micro-organisms which favourably influence the health of the host by improving the microbiological balance. Probiotic organisms must be:
- normal constituents of human flora or highly adaptable to that habitat;
- capable of specific adherence to the vaginal epithelium;
- easy to use in clinical practice.

Probiotic bacteria which meet that definition are mainly represented by lactic-acid-producing bacteria, and in particular lactobacilli (acidophilic). These live microbial agents are capable of rapid colonisation, which soon leads to performance of their functions:
1) protection by means of direct antagonism towards potentially pathogenic populations (inhibition of adherence to the epithelium; production of bacteriocins; competition for nutrients and substrates; creation of unfavourable pH conditions and redox microenvironments);
2) stimulation and education of the immune system (macrophagic activation, boosting of natural killer cells, increased production of interferons, and balancing of T- helper 1 and 2 populations).

The normal vaginal secretion has an acid pH of around 4.5; this acidity is due to the production of lactic acid from glycogen resulting from the action of Doderlein's bacillus (a particular lactobacillus). In order for a sufficient quantity of glycogen to be present in the vaginal epithelium, a normal level of oestrogens is required; consequently, at times of life when oestrogen production is low (pre-pubescent or post-menopausal age), the less acid vaginal pH makes colonisation by pathogenic germs easier. As local treatment based on oestrogens is not very practical, this problem can be tackled with "topical" use of lactic acid which, by reducing the pH to values of around 4, in itself constitutes a considerable physical barrier to colonisation by pathogens.

One of the active constituents of garlic extract, allicin, is known for its antimicrobial action against numerous bacteria, including *Staphylococcus aureus,* which is responsible for skin infections, and *Candida albicans,* which is responsible for yeast and thrush infections.

### DESCRIPTION OF THE INVENTION

This invention relates to topical vaginal formulations, preferably vaginal tablets or capsules, based on lactobacilli, lactic acid and garlic extract.

More particularly, the compositions according to the invention contain *Lactobacillus acidophilus,* lactic acid and garlic extract.

The compositions according to the invention provide a strong barrier to the development of common vaginal infections, and allow an ideal commensal microbial flora to be maintained in women of childbearing or menopausal age. The compositions according to the invention will contain *Lactobacillus acidophilus* in the quantity of between 100,000 and 20 billion per vaginal tablet or capsule, preferably 1 billion/vaginal tablet or capsule; lactic acid in the quantity of 1 to 50 mg, preferably 15 mg; and garlic extract in the quantity of 10 to 200 mg.

According to a preferred embodiment, the garlic extract will be present in the form of deodorised dried extract, containing alliin, allicin, aojene and allyl disulphide and trisulphide. According to an even more preferred embodiment, the garlic extract will be present in the form of deodorised dried extract titrated to a 1% allicin content.

According to this invention, the vaginal tablet or capsule is inserted deeply into the vagina, disintegrates within 5 minutes and releases the active constituents, thus restoring and stabilising the vaginal pH within a few minutes to acidity values of around 4-4.5, which are considered ideal to in protect against common infections. In the next few minutes, colonisation by lactobacilli takes place; they proliferate, aided by the immediate reduction in pH caused by the release of lactic acid, and restabilise the numerical ratios with the remaining portion of the commensal flora if necessary.

The consequences of this stabilisation are evident well-being of the vaginal area followed by a reduced risk of bacterial, fungal and mycoplasmal infections.

The topical vaginal formulation leads to the release of the extracted garlic derivatives which possess evident antiviral, bacteriostatic/antibacterial and antifungal properties.

The compositions according to the invention, which are administered vaginally, will be presented in the form of tablets, capsules, pessaries, ovules douches, creams and ointments prepared according to conventional methods well known in pharmaceutical technology, such as those described in "Remington's Pharmaceutical Handbook", Mack Publishing Co., N.Y., USA, together with suitable pharmaceutically acceptable excipients.

The following examples illustrate the invention in greater detail.

### Example 1

| **INGREDIENT** | **UNIT DOSE** |
|---|---|
| | **mg** |
| Deodorised dried garlic extract, titrated to a 1% allicin content | 100.000 |
| *Lactobacillus acidophilus* 150 billion CFU/g | 106.667 |
| 60% lactic acid | 15.000 |
| Mannitol CD | 908.333 |
| Explocel | 60.000 |
| Compritol E ATO | 70.000 |
| Levilite | 40.000 |
| Drygel Cerestar starch | 50.000 |
| **TOTAL** | **1350.000** |

### Example 2

| **INGREDIENT** | **UNIT DOSE** |
|---|---|
| | **mg** |
| Deodorised dried garlic extract, titrated to a 1% allicin content | 100.000 |
| *Lactobacillus acidophilus* 150 billion CFU/g | 106.667 |
| 60% lactic acid | 15.000 |
| Drygel Cerestar starch | 50.000 |
| Levilite | 10.000 |
| Lactose Pharmatose 200 M | 64.833 |
| Vegetable magnesium stearate | 3.500 |
| **TOTAL** | **350.000** |

## Claims

1. Topical vaginal pharmaceutical formulations **comprising effective concentrations of** lactobacilli, lactic acid and extract of garlic.

2. Compositions as claimed in claim 1, containing *Lactobacillus acidophilus,* lactic acid and dried garlic extract.

3. Compositions as claimed in claims 1 and 2, containing *Lactobacillus acidophilus* in the quantity of between 100,000 and 20 billion per vaginal tablet or capsule; 60% lactic acid in the quantity of 1 to 50 mg; and dried garlic extract in the quantity of 10 to 220 mg.

4. Compositions as claimed in the preceding claims, containing *Lactobacillus acidophilus* in the quantity of 1 billion/vaginal tablet or capsule; 60% lactic acid in the quantity of 15 mg; and dried garlic extract in the quantity of 100 mg.

5. Compositions as claimed in the preceding claims, wherein the garlic extract is present in the form of deodorised dried extract titrated to a 1% allicin content.

6. Compositions as claimed in the preceding claims, in the form of tablets, capsules, pessaries, ovules, douches, creams and ointments.

## Patentansprüche

1. Topische, vaginale pharmazeutische Formulierungen, umfassend effektive Konzentrationen an Laktobacillen, Milchsäure und Knoblauchextrakt.

2. Zusammensetzungen nach Anspruch 1, enthaltend *Lactobacillus acidophilus*, Milchsäure und getrocknetes Knoblauchextrakt.

3. Zusammensetzungen nach einem der Ansprüche 1 und 2, enthaltend *Lactobacillus acidophilus* in einer Menge von 100 000 bis 20 Mrd. pro vaginaler Tablette oder Kapsel; 60 % Milchsäure in einer Menge von 1 bis 50 mg; und getrocknetes Knoblauchextrakt in einer Menge von 10 bis 220 mg.

4. Zusammensetzungen nach einem der vorherigen Ansprüche, enthaltend *Lactobacillus acidophilus* in einer Menge von 1 Mrd./vaginaler Tablette oder Kapsel; 60 % Milchsäure in einer Menge von 15 mg; und getrocknetes Knoblauchextrakt in einer Menge von 100 mg.

5. Zusammensetzungen nach einem der vorherigen Ansprüche, worin das Knoblauchextrakt in Form eines desodorierten getrockneten Extraktes, welches auf einen 1 % Allicin-Gehalt titriert wurde, vorliegt.

6. Zusammensetzungen nach einem der vorherigen Ansprüche in Form von Tabletten, Kapseln, Pessaren, Ovulen, Spülungen, Cremes und Salben.

## Revendications

1. Formulations pharmaceutiques topiques vaginales comprenant des concentrations efficaces de lactobacilles, d'acide lactique et d'extrait d'ail.

2. Compositions selon la revendication 1, contenant du *Lactobacillus acidophilus,* de l'acide lactique et de l'extrait d'ail séché.

3. Compositions selon les revendications 1 et 2, contenant du *Lactobacillus acidophilus* en la quantité comprise entre 100 000 et 20 milliards par comprimé ou capsule vaginal(e) ; 60 % d'acide lactique en la quantité de 1 à 50 mg ; et de l'extrait d'ail séché en la quantité de 10 à 220 mg.

4. Compositions selon l'une quelconque des revendications précédentes, contenant du *Lactobacillus acidophilus* en la quantité de 1 milliard/comprimé ou capsule vaginal(e) ; 60 % d'acide lactique en la quantité de 15 mg ; et de l'extrait d'ail séché en la quantité de 100 mg.

5. Compositions selon l'une quelconque des revendications précédentes, dans lesquelles l'extrait d'ail est présent sous la forme d'un extrait sec désodorisé titré à une teneur en allicine de 1 %.

6. Compositions selon l'une quelconque des revendications précédentes, sous la forme de comprimés, capsules, pessaires, ovules, lavages internes, crèmes et pommades.
